# FASCICULE DE BREVET EUROPEEN

(11) **EP 1 442 758 B1**
(45) Date de publication et mention de la délivrance du brevet: **15.08.2007**
(21) Numéro de dépôt: 03293279.0
(22) Date de dépôt: 22.12.2003
(51) Int. Cl.: A61M 1/02, A61M 1/36

(54) **Machine de prélèvement de fluide biologique auquel est ajoutée une solution selon un ratio désiré**
Vorrichtung zur Entnahme einer biologischen Flüssigkeit, der eine Lösung in einem gewünschten Verhältnis zugefügt wird
Apparatus for extraction of biological a fluid to which a solution is added at a desired ratio

(30) Priorité: 03.02.2003 FR 0301195
(43) Date de publication de la demande: 04.08.2004
(73) Titulaire: MACO PHARMA, 59420 Mouvaux (FR)
(72) Inventeur: Behague, Maurice, 59126 Linselles (FR); Goudaliez, Francis, 59155 Faches-Thumesnil (FR); Verpoort, Thierry, 59420 Mouvaux (FR)
(74) Mandataire: Geismar, Thierry

(56) Documents cités:
- EP-A- 0 583 148
- FR-A- 2 808 693
- US-A- 4 258 723
- US-A- 4 267 837
- US-A- 6 113 554

## Description

L'invention concerne une machine de prélèvement d'un fluide biologique additionné d'une solution anticoagulante et/ou de conservation et un système à poches pour le prélèvement d'un fluide biologique au moyen d'une telle machine.

Elle s'applique typiquement au cas où le fluide biologique est du sang total qui doit être prélevé d'un donneur dans une poche de recueil. En effet, il est recommandé que le sang soit prélevé de façon stérile et soit additionné d'une solution anticoagulante et/ou de conservation lors du prélèvement de sorte à permettre son utilisation ultérieure dans les meilleures conditions de sécurité sanitaire.

Pour ce faire, il est connu, préalablement au prélèvement, de remplir la poche de recueil avec la solution anticoagulante et/ou de conservation, et de remplir ensuite la poche de recueil en fluide lors du prélèvement.

Un des problèmes qui se pose est alors que, pour essayer de réaliser un mélange homogène entre le fluide et la solution contenue dans la poche, il est nécessaire d'agiter la poche de recueil, ce qui complique le procédé de prélèvement et ne donne pas entièrement satisfaction.

Un autre problème qui se pose concerne l'obtention d'un ratio déterminé entre la quantité de fluide prélevé et la quantité de solution anticoagulante et/ou de conservation additionnée. En effet, notamment dans le domaine du prélèvement sanguin, la quantité de solution anticoagulante et/ou de conservation présente dans la poche de recueil est fixée à une certaine valeur pour que le sang soit utilisable dans le domaine médical.

Et, lorsque la solution anticoagulante et/ou de conservation est présente dans la poche de recueil préalablement au prélèvement, le ratio est correct uniquement pour une quantité de fluide prélevé donnée. Mais, ce ratio est considérablement plus élevé au début du prélèvement ou lorsque le prélèvement est interrompu avant que toute la quantité de fluide souhaitée soit prélevée. Dans le cas du sang, il peut en résulter une baisse de la qualité du sang par lyse des globules rouges et détérioration de la fonctionnalité des plaquettes, voir une impossibilité d'utiliser le sang prélevé.

Pour résoudre ce problème, il a été proposé, notamment par les documents FR-2 808 693 et US-6 113 554, de placer la solution anticoagulante et/ou de conservation dans une poche séparée de la poche de recueil, et d'alimenter la poche de recueil en solution simultanément au prélèvement du fluide.

Le document EP-0583148 décrit une machine de prélèvement selon le préambule de la revendication 1.

Ces solutions ne donnent pas entièrement satisfaction notamment à deux niveaux.

Le premier problème concerne l'obtention du ratio entre la quantité de fluide prélevé et la quantité de solution anticoagulante et/ou de conservation additionnée. En effet, dans ces documents, le ratio est obtenu soit en utilisant un système à poches spécifique soit en utilisant une structure de pompe complexe, ce qui complique leur utilisation et augmente sensiblement le coût du prélèvement.

Le deuxième problème est relatif au pompage du fluide qui, en imposant un flux de fluide prélevé, est nécessaire pour obtenir le ratio. En effet, pour des raisons de sécurité et dans le cas du prélèvement du sang d'un donneur, il est alors souhaitable d'utiliser un capteur de pression du fluide en amont de la pompe, afin de prévenir tout risque de collabage de la veine du donneur. Ce capteur, outre sa complexité et son coût, peut engendrer des problèmes d'asepsie s'il est basé sur un échange gazeux entre l'intérieur et l'extérieur du système de prélèvement. En outre, le pompage est source d'inconfort et d'insécurité pour le donneur.

L'invention vise à résoudre l'ensemble de ces problèmes en proposant notamment un procédé de prélèvement particulièrement simple de mise en oeuvre, qui est confortable et sûr pour le donneur et qui permet d'obtenir en continu lors du prélèvement un ratio déterminé entre la quantité de fluide prélevé et la quantité de solution anticoagulante et/ou de conservation additionnée.

Selon un premier aspect, l'invention concerne une machine de prélèvement selon la revendication 1.

Selon une réalisation, le dispositif de mesure comprend un moyen de mesure du poids et de calcul de la variation du poids de fluide prélevé, et le dispositif de pompage comprend un pompe péristaltique à une seule tête qui est mobile en rotation à vitesses variables.

Selon un second aspect, l'invention concerne un système à poches selon la revendication 4.

Selon une réalisation, le système à poches peut être dépourvu de moyen permettant de mesurer la pression à l'intérieur dudit système.

D'autres objets et avantages de l'invention apparaîtront au cours de la description qui suit en référence aux dessins annexés.

La figure 1 représente de façon schématique le fonctionnement d'une machine de prélèvement selon une première réalisation de l'invention, dans laquelle est disposé un système à poches.

La figure 2 représente de façon schématique le fonctionnement d'une machine de prélèvement selon une deuxième réalisation de l'invention, dans laquelle est disposé un système à poches.

La figure 3 représente de façon schématique un système à poches disposé sur le dispositif de placement d'une machine de prélèvement selon l'invention.

La figure 4a représente une vue en perspective avant de la machine de prélèvement.

La figure 4b représente une vue en perspective arrière de la machine de prélèvement.

La figure 5 représente un organigramme relatif au fonctionnement de la machine de prélèvement pour l'obtention du ratio déterminé entre la quantité de fluide prélevé et la quantité de solution anticoagulante et/ou de conservation additionnée.

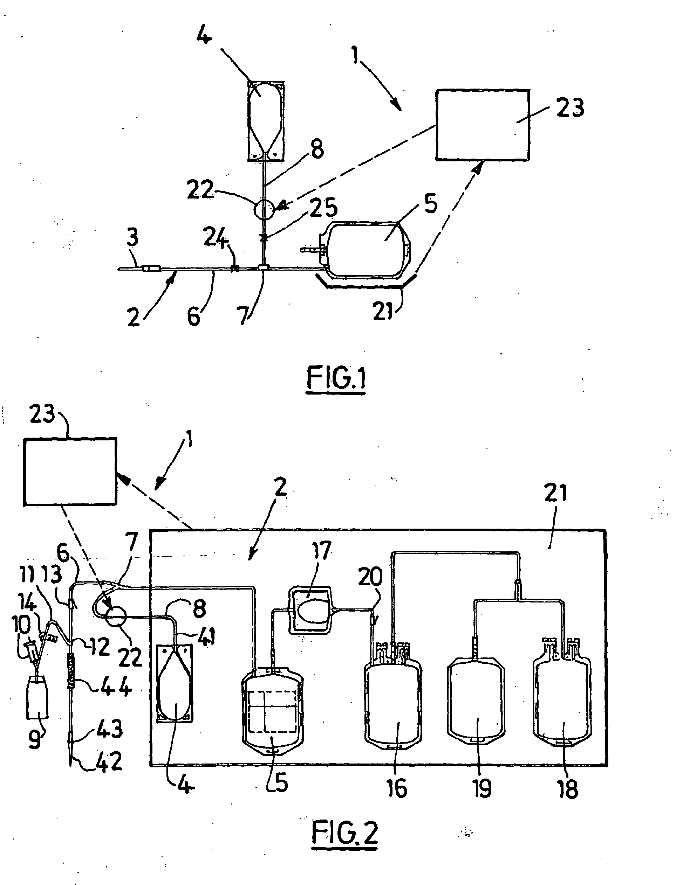

La figure 1 représente un système à poches 2 comprenant des moyens de prélèvement 3 du fluide auprès d'un patient, au moins une poche 4 contenant une solution anticoagulante et/ou de conservation du fluide prélevé, et au moins une poche de recueil 5 destinée à recevoir le fluide prélevé additionné de la solution anticoagulante et/ou de conservation.

Par exemple, le système à poches 2 est stérilisé et conditionné dans un emballage stérile.

Les moyens de prélèvement 3 sont constitués notamment d'une aiguille 42 permettant l'accès à la veine du donneur et d'un capuchon 43 protégeant l'aiguille 42. En outre, un protecteur d'aiguille 44 peut être placé de façon coulissante sur une première tubulure 6.

La poche de recueil 5 est en communication fluidique avec les moyens de prélèvement 3 par l'intermédiaire de la première tubulure 6 souple. La poche 4 contenant une solution anticoagulante et/ou de conservation est en communication fluidique avec la poche de recueil 5 par l'intermédiaire d'une deuxième tubulure 8 souple reliée au niveau d'un connecteur 7 à la première tubulure 6. Ce connecteur est une jonction trois voies sur laquelle sont branchées, d'une part, une première partie de la première tubulure 6 en provenance des moyens de prélèvement 3 et la deuxième tubulure 8 et d'autre part une deuxième partie de la première tubulure 6 en direction de la poche de recueil 5.

Selon une réalisation, la partie de la première tubulure 6 comprise entre le connecteur 7 et la poche de recueil 5 est de longueur suffisante pour obtenir un mélange homogène entre le fluide prélevé et la solution anticoagulante et/ou de conservation, lorsque le mélange atteint l'orifice d'entrée de la poche de recueil 5. La longueur de cette partie de la première tubulure 6 est notamment supérieure à 15 cm, par exemple de l'ordre de 25 cm.

La figure 2 représente un système à poches 2 comprenant, outre le système à poches représenté sur la figure 1, une poche d'échantillonnage 9 destinée à recevoir les premiers millilitres de sang prélevé, un dispositif d'échantillonnage latéral 10 associé à ladite poche d'échantillonnage 9 de sorte à permettre le prélèvement d'échantillons à l'aide de tubes sous vide. La poche d'échantillonnage 9 est en communication fluidique avec la poche de recueil 5 par l'intermédiaire de deux tubulures 6, 11 souples reliées au niveau d'un connecteur 12 sous la forme d'une jonction trois voies.

Le remplissage de la poche d'échantillonnage 9 et de la poche de recueil 5 résulte de l'écoulement naturel, c'est à dire la gravité et la pression veineuse du donneur. Un écoulement naturel basé sur la pression veineuse et la gravité offre un confort et une sécurité supérieurs au donneur.

Des clamps 13, 14 peuvent être situés respectivement sur la première tubulure 6 souple, en aval du connecteur 12, et sur la tubulure 11 souple. Lesdits clamps 13, 14 permettent d'orienter, vers la poche d'échantillonnage 9, les premiers millilitres de fluide prélevés, le clamp 13 est alors fermé pendant que le clamp 14 est ouvert. Lorsque la poche d'échantillonnage 9 est pleine, on ferme le clamp 14 et on ouvre le clamp 13 afin d'orienter le fluide prélevé vers la poche de recueil 5.

Des ouvre-circuits peuvent être prévus au niveau du connecteur 12 ainsi qu'au niveau des orifices d'entrée et de sortie des poches contenant du sang ou des composants du sang. Un ouvre-circuit 41 est notamment disposé sur la deuxième tubulure, au niveau de son extrémité en communication fluidique avec la poche 4 contenant la solution anticoagulante et/ou de conservation, et ce afin d'éviter une remontée de la solution anticoagulante et/ou de conservation lors de la stérilisation du système, notamment par vapeur d'eau.

Par ailleurs, il est préférable que l'ouvre-circuit ne se trouve pas sur le passage d'écoulement du sang compris entre les moyens de prélèvement 3 et la poche de recueil 5 afin de prévenir tout risque d'hémolyse du sang prélevé au moment du prélèvement.

Afin de réaliser des étapes de filtration et de séparation des différents constituants du sang, la poche de recueil 5 peut être en communication fluidique, par l'intermédiaire d'une quatrième tubulure 15 souple, avec une poche satellite 16. Un filtre à déleucocyter 17 est situé entre la poche de recueil 5 et la poche satellite 16.

La poche satellite 16 peut être en communication fluidique avec une ou plusieurs autres poches satellites, par exemple la poche satellite 16 peut être en communication fluidique avec deux autres poches satellites 18, 19.

Un clamp 20 peut être prévu sur la tubulure 15 souple entre la poche de recueil 5 et le filtre à déleucocyter 17.

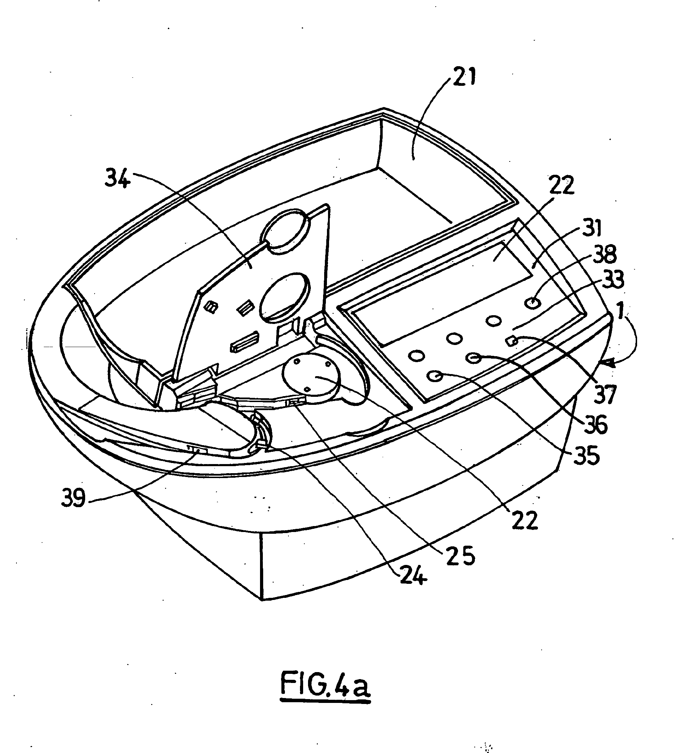

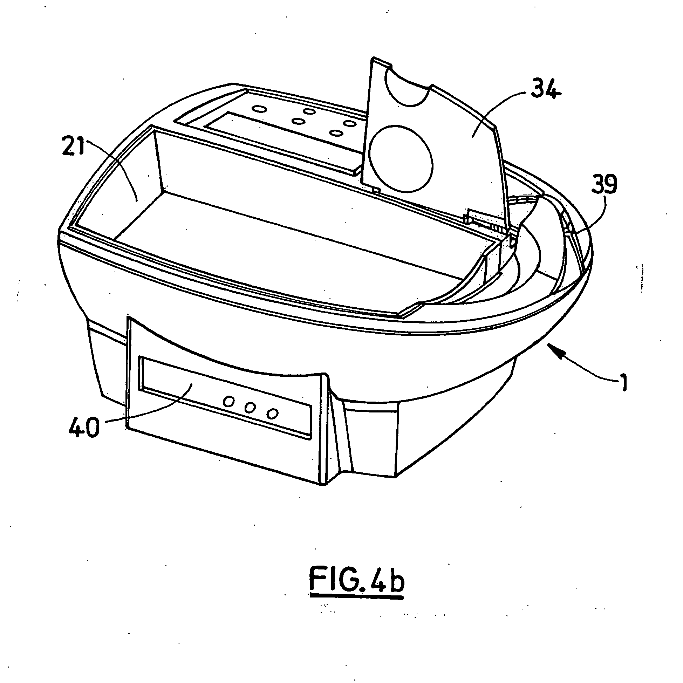

Le système à poches décrit ci-dessus est destiné à être disposé dans le dispositif de placement d'une machine de prélèvement telle que représentée sur les figures 4a et 4b.

La figure 4a est une vue en perspective de la face avant de la machine de prélèvement 1. Elle comprend le dispositif de pesage 21, d'une dimension suffisamment importante pour accueillir un système à poches 2 tel que décrit ci-dessus.

La machine de prélèvement comprend en outre un dispositif de pompage 22 comprenant une pompe péristaltique à une seule tête qui est mobile en rotation à vitesses variables pour pomper la solution anticoagulante et/ou de conservation, le prélèvement du fluide résultant de l'écoulement naturel, c'est à dire la gravité et la pression veineuse du donneur. Ainsi, le système à poches 2 peut être dépourvu de moyen permettant de mesurer la pression à l'intérieur dudit système.

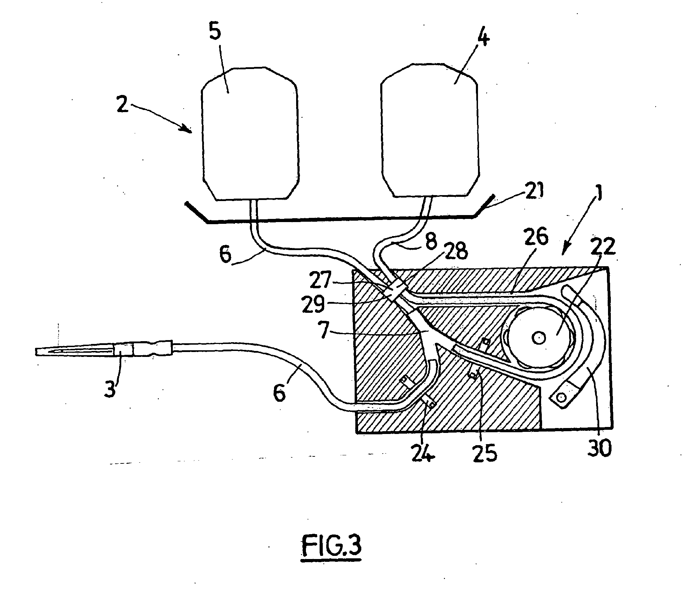

Représenté sur les figures 1 et 2, le dispositif de pesage 21 permet de connaître instantanément le poids et les variations de poids du fluide prélevé et ainsi le débit de fluide prélevé. Pour cela on place sur ce dispositif de pesage 21 l'ensemble du système à poches 2 comme représenté sur les figures 2 et 3, une tarre est effectuée par le dispositif de pesage 21 après le placement du système à poches 2, afin de tenir compte du poids dudit système à poches 2 avant prélèvement. Après une courte période négligeable (correspondant à la circulation de la solution dans les tubulures 6, 8), les variations de poids déterminées par le dispositif de pesage 21 sont ainsi uniquement corrélées au flux du fluide prélevé. En effet, l'anticoagulant passe de la poche 4 contenant la solution anticoagulante et/ou de conservation à la poche 5 de recueil, ces deux poches 4, 5 étant sur le dispositif de pesage 21, la circulation de la solution n'entraîne pas de variations de poids.

La pompe péristaltique 22 comprend une tête d'écrasement autour de laquelle est disposée une partie de la deuxième tubulure 8 souple, permettant d'asservir le débit de la solution par rapport au calcul des variations de poids du fluide prélevé, par l'intermédiaire d'une électronique de pilotage du moteur de la pompe 23.

L'électronique de pilotage 23 transcrit les calculs de variations de poids en débit de fluide prélevé, par un calcul tenant compte de la densité du fluide prélevé. L'électronique de pilotage 23 détermine ensuite le débit de la solution anticoagulante et/ou de conservation à fixer, selon le ratio déterminé, et elle règle la vitesse de rotation de la pompe péristaltique 22 pour alimenter, selon ce débit déterminé, la poche 5 de recueil en solution anticoagulante et/ou de conservation.

Selon un mode de réalisation représenté figure 1, Il est également envisageable de placer l'ensemble du système à poches sauf la poche 4 contenant la solution anticoagulante et/ou de conservation sur le dispositif de pesage 21, comme représenté sur la figure 1, mais il faut alors tenir compte, en plus de la tarre normalement effectuée, d'une constante qui est la variation de poids liée à l'introduction de solution anticoagulante et/ou de conservation dans ladite poche de recueil 5.

Selon un mode particulier de l'invention, des capteurs optiques peuvent être ajoutés à la machine 1. Un premier capteur optique 24 peut être placé sur la première tubulure 6 entre les moyens de prélèvement 3 et le connecteur 7, de préférence entre les connecteurs 7 et 12. Ce capteur 24 permet de détecter la présence de sang, afin de vérifier que le sang circule convenablement au sein de la première tubulure 6. II permet également de vérifier s'il n'y a pas d'air ou de solution anticoagulante et/ou de conservation remontant vers les moyens de prélèvement 3, donc vers le donneur. Ce capteur optique peut par exemple être remplacé ou complété par un capteur à ultrasons permettant de détecter de façon plus fine les inversions de flux.

Un deuxième capteur optique 25 peut être placé sur la deuxième tubulure 8 entre la tête de la pompe péristaltique 22 et le connecteur 7. Ce capteur 25 permet de détecter la présence de solution anticoagulante et/ou de conservation, afin de vérifier que la solution anticoagulante et/ou de conservation circule convenablement au sein de la deuxième tubulure 8.

La machine 1 comprend en outre un dispositif de placement prévu pour recevoir le système à poches 2 ainsi que représenté sur la figure 3. Le dispositif de placement comprend une gorge 26 dans laquelle l'utilisateur place les tubulures 6, 8, un dispositif courbe 30 pouvant être prévu pour appuyer la deuxième tubulure 8 sur la pompe péristaltique 22 afin de permettre un fonctionnement correct de celle-ci. La machine 1 comprend en outre un clamp automatique 39 et un capot 34 qui protége et assure un maintien optimal du placement du système à poches dans la gorge 26 de la machine 1. Le dispositif courbe 30 prévu pour appuyer la deuxième tubulure 8 sur la pompe péristaltique 22 s'écarte de la deuxième tubulure 8 lorsque le capot 34 est ouvert.

Lorsque la pompe péristaltique 22 est en mouvement, les galets de la tête de pompe 22 viennent comprimer successivement la section de la deuxième tubulure 8 contre le dispositif courbe 30 de façon à assurer le déplacement de la solution anticoagulante et/ou de conservation dans la deuxième tubulure.

La machine de prélèvement 1 comprend, en outre, une interface 31 qui se divise en deux zones, un afficheur 32 et un clavier 33, comme représenté sur la figure 4a. L'afficheur 32 permet de donner à l'utilisateur de la machine des informations diverses : il affiche des informations générales comme la date, l'heure ou encore le niveau des batteries; des informations concernant le prélèvement en cours, le numéro du prélèvement, le volume prélevé, la durée du prélèvement ou encore le volume de sang désiré ; ainsi que des messages d'erreurs lorsque le débit du fluide prélevé n'est pas correct ou encore lorsque le capot 34 de la machine 1 n'est pas fermé.

Le clavier 33 comprend un certain nombre de touches ayant des fonctions diverses. La touche veille 35 permet d'allumer et d'éteindre la machine de prélèvement 1. La touche stop 36 permet d'arrêter le prélèvement en cas de d'urgence. Le voyant alarme 37 s'allume pour prévenir l'utilisateur d'un mauvais fonctionnement, par exemple lorsque le débit du fluide prélevé n'est pas situé dans la plage autorisée, dont les valeurs sont comprises entre 30 et 350 millilitres par minute, notamment entre 50 et 250 millilitres par minute. Les touches de navigation 38 permettent à l'utilisateur de naviguer dans les différents menus proposés par la machine 1, les menus principaux étant la modification des paramètres, la réalisation d'un autotest et la réalisation d'un prélèvement.

La machine 1 peut, en outre, comprendre des connecteurs prévus au niveau de la zone 40 pour l'entrée et la sortie de données vers une imprimante, un lecteur code barres ou encore un micro-ordinateur, comme cela est représenté sur la figure 4b.

Le placement du système à poches 2 dans le dispositif de placement de la machine 1, ainsi que représenté figure 3, peut être facilité par la formation d'une boucle, lors de la fabrication dudit système, en associant la première tubulure 6 et la deuxième tubulure 8, à l'aide d'un moyen d'association 27. Ce moyen d'association est constitué d'un connecteur droit 28 sous la forme d'un logement cylindrique dans lequel on insert la deuxième tubulure 8, et d'un clip latéral 29 en forme de U dans lequel la tubulure 6 est clipsée permettant ainsi une fixation de la première tubulure 6 à la deuxième tubulure 8.

Pour assurer une fixation des deux tubulures 6 et 8 l'une à l'autre, l'assemblage des tubulures et du moyen d'association 27 est réalisé, par exemple, par collage par solvant.

La boucle ainsi formée permet de faciliter le positionnement du système à poches sur la machine de prélèvement 1 et fait office de détrompeur, puisque, de ce fait, celle-ci ne peut se positionner que dans un sens dans le dispositif de placement.

Cette boucle permet également d'empêcher l'entraînement de la deuxième tubulure 8 par la pompe péristaltique 22, puisque le moyen d'association 27 et la jonction trois voies 7 sont bloqués dans des logements prévus à cet effet dans la gorge 26 de la machine de prélèvement 1, que le connecteur droit est intégré à la deuxième tubulure 8 et que les deux tubulures 6 et 8 sont accrochées l'une à l'autre. Lorsqu'une tubulure est entrainée par la rotation de la pompe péristaltique, la circulation du fluide au sein de cette tubulure est perturbée. L'immobilisation de la deuxième tubulure 8 permet donc un fonctionnement optimal de la pompe péristaltique 22.

Selon une réalisation, la partie de la deuxième tubulure 8, qui est située entre le connecteur 7 et le moyen d'association 26, possède des caractéristiques physiques appropriées afin de bien maîtriser le débit créé par ladite pompe péristaltique 22.

Par exemple, la partie de la deuxième tubulure 8 formant la boucle peut présenter une dureté inférieure à celle de la première tubulure 6. En particulier, la deuxième tubulure 8 possède une dureté comprise entre 60 et 70 shore A, notamment 65 shore A. Les autres tubulures ont une dureté supérieure à 70 shore A, notamment 78.

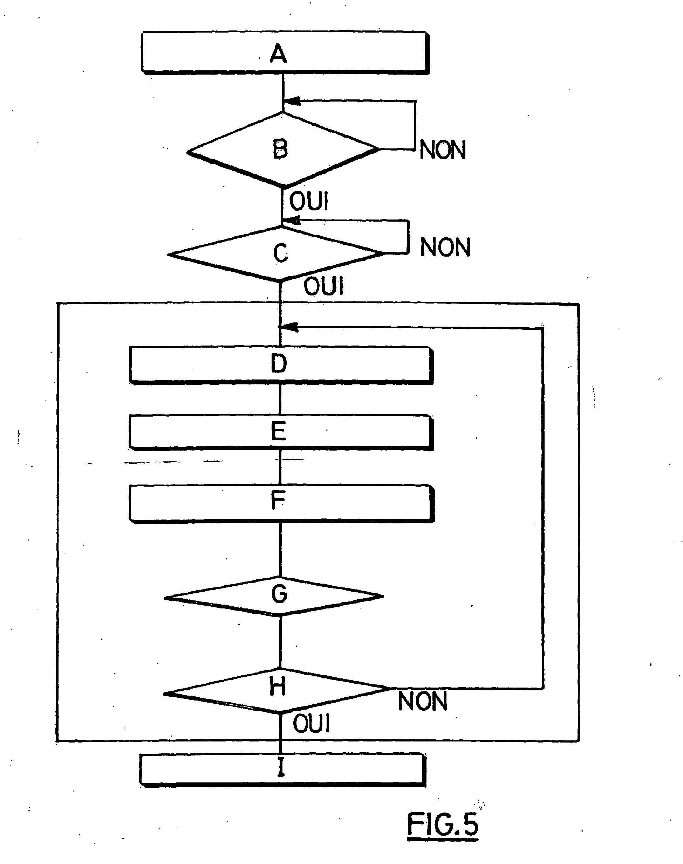

La figure 5 est un organigramme relatif au fonctionnement de la machine de prélèvement pour l'obtention du ratio désiré entre la quantité de sang prélevé et la quantité de solution anticoagulante et/ou de conservation additionnée.

Une séquence de prélèvement réalisée selon le procédé de l'invention et utilisant la machine de prélèvement 1 va maintenant être décrite.

Pour mettre en marche la machine de prélèvement 1, l'utilisateur appuie sur la touche veille 35. A l'aide des touches de navigation 38, l'utilisateur choisit ensuite le menu principal relatif à la réalisation d'un prélèvement. L'afficheur 32 indique qu'il est possible de procéder à l'installation du système à poches 2. Puis, l'utilisateur ferme le capot 34. Par l'intermédiaire des informations recueillies par les capteurs 24, 25, l'afficheur 32 précise si le système à poches 2 est correctement placé.

Avant de démarrer le prélèvement proprement dit, la machine 1 réalise une étape d'initialisation du prélèvement (A).

La machine 1 fait circuler la solution anticoagulante jusqu'au connecteur 7, ceci permet de prélever le sang sans risque de coagulation. Le capteur 25 permet de détecter la présence de solution anticoagulante et/ou de conservation, afin de vérifier que celle-ci a atteint le connecteur 7. Il est possible de faire faire à la pompe péristaltique 22 au moins une rotation supplémentaire après que le capteur 25 a détecté la présence de la solution, de manière à ce que la solution anticoagulante et/ou de conservation soit présente au sein de la première tubulure 6 avant que le sang n'y circule.

Durant cette étape (A), l'utilisateur introduit les moyens de prélèvement 3 dans la veine du donneur, puis, procède à la prise d'échantillons par l'intermédiaire du dispositif d'échantillonnage latéral 10 associé à la poche d'échantillonnage 9 et des clamps 13, 14.

Lorsque cette étape (A) est réalisée correctement, l'afficheur 32 indique que le prélèvement peut être lancé.

L'étape de lancement du prélèvement (B) commence par l'ouverture du clamp automatique 39, lorsque l'utilisateur appuie sur la touche de navigation 38 correspondante.

L'étape de détection du sang (C) est réalisée par l'intermédiaire du capteur optique 24. Ce capteur optique 24 permet de détecter la présence de sang, afin de vérifier que le sang est présent au sein de la première tubulure 6 au niveau de ce capteur 24. On peut ainsi estimer que le fluide circule convenablement au sein de la première tubulure 6.

A partir de l'étape (D), la machine 1 met en oeuvre le dispositif de pesage 21, la pompe péristaltique 22 et l'électronique de pilotage 23 afin d'obtenir un ratio désiré entre la quantité de sang prélevé et la quantité de solution anticoagulante et/ou de conservation additionnée.

L'étape de calcul de la variation de poids (D) est réalisée par le dispositif de pesage 21. L'étape (E) de calcul du débit de sang est réalisée à partir des résultats obtenus pendant l'étape (D). Les étapes (F) et (G) concernent respectivement l'ajustement de la vitesse de rotation de la pompe péristaltique 22 et la vérification de la valeur du débit de sang. Concernant l'ajustement de la vitesse de la pompe péristaltique 22, l'électronique de pilotage 23 calcule, à partir du débit de sang obtenu à l'étape (E), le débit d'anticoagulant à obtenir. En particulier, le ratio entre la quantité de solution anticoagulante et/ou de conservation et celle de sang peut être fixé à 1/7. L'électronique de pilotage 23 transcrit ensuite ce débit d'anticoagulant en une certaine vitesse de rotation de la pompe péristaltique 22. La fréquence d'ajustement de la vitesse de rotation de la pompe péristaltique 22 est telle que la période entre deux ajustements est inférieure à la seconde, plus particulièrement de l'ordre du dixième de seconde.

L'étape (H) concernant l'obtention du volume programmé de sang est réalisée par le dispositif de pesage 21. Le volume programmé correspond à un certain poids, lorsque le dispositif de pesage 21 mesure ce poids, le prélèvement s'arrête. Tant que ce poids n'est pas atteint, la machine 1 continue la boucle constituée par les étapes (D) à (G).

L'étape I marque la fin du prélèvement.

Le volume de sang prélevé est variable selon les zones géographiques. Un avantage du dispositif selon l'invention est que l'on peut choisir le volume de sang prélevé au moment de la collecte, et qu'il n'est pas nécessaire de prévoir des systèmes à poches différents contenant différents volumes d'anticoagulant. Par ailleurs, au cas où l'utilisateur doit interrompre le prélèvement avant la fin programmée du prélèvement, en appuyant sur la touche stop 36, le sang est utilisable puisque le ratio entre la quantité de sang prélevé et celle de solution anticoagulante et/ou de conservation est correct tout au long du prélèvement.

## Revendications

1. Machine pour le prélèvement (1) d'un fluide biologique, notamment sanguin, additionné d'une solution anticoagulante et/ou de conservation comprenant un dispositif de mesure du débit de fluide prélevé (21) et un dispositif de pompage (22) de la solution anticoagulante et/ou de conservation, ledit dispositif de pompage comprenant un moyen d'asservissement de la vitesse de pompage en fonction de la valeur du débit de fluide qui est issue du dispositif de mesure (21), ladite machine étant **caractérisée en ce qu**'elle est dépourvue de moyen de pompage du fluide biologique qui est prélevé par écoulement naturel.

2. Machine selon la revendication 1, **caractérisée en ce que** le dispositif de mesure (21) comprend un moyen de mesure du poids et de calcul de la variation du poids de fluide prélevé.

3. Machine selon la revendication 1 ou 2, **caractérisée en ce que** le dispositif de pompage (22) comprend une pompe péristaltique à une seule tête qui est mobile en rotation à vitesses variables.

4. Système à poches (2) pour le prélèvement d'un fluide biologique au moyen d'une machine (1) selon la revendication 3, ledit système comprenant, en circuit clos, des moyens de prélèvement du fluide (3), une poche (4) contenant une solution anticoagulante et/ou de conservation du fluide prélevé, et une poche de recueil (5) destinée à recevoir le fluide prélevé additionné de la solution anticoagulante et/ou de conservation, dans lequel la poche de recueil (5) est en communication fluidique avec les moyens de prélèvement (3) par l'intermédiaire d'une première tubulure (6) souple et avec la poche (4) contenant la solution anticoagulante et/ou de conservation par l'intermédiaire d'une deuxième tubulure (8) souple, ledit système étant **caractérisé en ce qu**'au moins la partie de la deuxième tubulure (8) qui est destinée à être écrasée par la tête de la pompe péristaltique (22) présente une dureté inférieure à celle de la première tubulure (6).

5. Système selon la revendication 4, **caractérisé en ce qu**'il est dépourvu de moyen permettant de mesurer la pression à l'intérieur dudit système.

6. Système selon la revendication 4 ou 5, **caractérisé en ce que** la deuxième tubulure (8) est pourvue d'un ouvre circuit (41) qui est disposé à proximité de son extrémité en communication fluidique avec la poche (4) contenant la solution anticoagulante et/ou de conservation.

7. Système selon l'une quelconque des revendications 4 à 6, **caractérisé en ce que** la deuxième tubulure (8) est branchée sur la première tubulure (6) au moyen d'un connecteur (7).

8. Système selon la revendication 7, **caractérisé en ce que** la longueur de la première tubulure (6) mesurée entre le connecteur (7) et l'orifice d'entrée de la poche de recueil (5) est supérieur à 15 cm, notamment de l'ordre de 25 cm.

## Claims

1. Machine for sampling (1) a biological fluid, in particular blood, with an added anticoagulant and/or preservative solution, comprising a device (21) for measuring the flow of sampled fluid and a device (22) for pumping the anticoagulant and/or preservative solution, said pumping device comprising means for adjusting the pumping speed according to the value of the flow of fluid coming from the measuring device (21), said machine being **characterised in that** it lacks means for pumping the biological fluid, which is sampled by natural discharge.

2. Machine according to claim 1, **characterised in that** the measurement device (21) comprises means for measuring the weight and calculating the variation in the weight of the sampled fluid.

3. Machine according to claim 1 or 2, **characterised in that** the pumping device (22) comprises a peristaltic pump with a single head, which is rotatingly mobile at variable speeds.

4. System of bags (2) for sampling a biological fluid by means of a machine (1) according to claim 3, said system comprising, in a closed circuit, means for sampling the fluid (3), a bag (4) containing an anticoagulant and/or preservative solution for the sampled fluid, and a receiving bag (5) designed to receive the sampled fluid with the added anticoagulant and/or preservative solution, wherein the receiving bag (5) is in fluidic communication with the sampling means (3) by means of a first flexible tube (6) and with the bag (4) containing the anticoagulant and/or preservative solution by means of a second flexible tube (8), said system being **characterised in that** at least the part of the second tube (8) designed to be squeezed by the head of the peristaltic pump (22) has a hardness that is less than that of the first tube (6).

5. System according to claim 4, **characterised in that** it lacks means for measuring the pressure inside said system.

6. System according to claim 4 or 5, **characterised in that** the second tube (8) is equipped with a circuit opener (41) arranged near its end in fluidic communication with the bag (4) containing the anticoagulant and/or preservative solution.

7. System according to any one of the claims from 4 to 6, **characterised in that** the second tube (8) is connected to the first tube (6) by means of a connector (7).

8. System according to claim 7, **characterised in that** the length of the first tube (6) measured between the connector (7) and the intake orifice of the receiving bag (5) is greater than 15 cm, in particular around 25 cm.

## Patentansprüche

1. Maschine für die Entnahme (1) eines biologischen Fluids, insbesondere von Blut, mit Zusatz einer blutgerinnungshemmenden und/oder Konservierungslösung, die eine Vorrichtung für die Messung der Abflussmenge des entnommenen Fluids (21) und eine Vorrichtung für das Pumpen (22) der blutgerinnungshemmenden und/oder Konservierungslösung umfasst, wobei die besagte Pumpvorrichtung ein Steuermittel der Pumpgeschwindigkeit in Abhängigkeit vom Wert der Abflussmenge des Fluids umfaßt, der aus der Messvorrichtung (21) hervorgegangen ist, wobei die besagte Maschine **dadurch gekennzeichnet ist, daß** sie kein Mittel zum Pumpen des biologischen Fluids hat, das durch natürliches Abfließen entnommen wird.

2. Maschine nach Anspruch 1, **dadurch gekennzeichnet, daß** die Messvorrichtung (21) ein Mittel zur Messung des Gewichts und der Berechnung der Gewichtsschwankung des entnommenen Fluids umfaßt.

3. Maschine nach Anspruch 1 oder 2, **dadurch gekennzeichnet, daß** die Pumpvorrichtung (22) eine Peristaltikpumpe mit einem einzigen Kopf hat, der in Drehung bei variablen Geschwindigkeiten mobil ist.

4. Beutelsystem (2) für die Entnahme eines biologischen Fluids vermittels einer Maschine (1) nach Anspruch 3, wobei das besagte System im geschlossenen Kreislauf Mittel zur Entnahme des Fluids (3) umfaßt, einen Beutel (4) mit einer blutgerinnungshemmenden und/oder Konservierungslösung des entnommenen Fluids und einen Sammelbeutel (5), der für die Aufnahme des entnommenen Fluids mit Zugabe der blutgerinnungshemmenden und/oder Konservierungslösung bestimmt ist, in dem der Sammelbeutel (5) in Fluidverbindung mit den Mitteln zur Entnahme (3) über einen ersten elastischen Stutzen (6) und mit dem Beutel (4) steht, der die blutgerinnungshemmende und/oder Konservierungslösung enthält, über einen zweiten elastischen Stutzen (8), wobei das besagte System **dadurch gekennzeichnet ist, daß** zumindest der Teil des zweiten Stutzens (8), der durch den Kopf der Peristaltikpumpe (22) zerstört werden soll, eine geringere Härte als diejenige des ersten Stutzens (6) aufweist.

5. System nach Anspruch 4, **dadurch gekennzeichnet, daß** es kein Mittel hat, mit dem der Druck im Innern des besagten Systems gemessen werden kann.

6. System nach Anspruch 4 oder 5, **dadurch gekennzeichnet, daß** der zweite Stutzen (8) mit einem Kreislauföffner (41) versehen ist, der in der Nähe seines Endes angeordnet ist, das in Fluidverbindung mit dem Beutel (4) steht, der die blutgerinnungshemmende und/oder Konservierungslösung enthält.

7. System nach einem beliebigen der vorstehenden Ansprüche 4 bis 6, **dadurch gekennzeichnet, daß** der zweite Stutzen (8) vermittels eines Verbinders (7) an den ersten Stutzen (6) angeschlossen ist.

8. System nach Anspruch 7, **dadurch gekennzeichnet, daß** die zwischen dem Verbinder (7) und der Eintrittsöffnung des Sammelbeutels (5) gemessene Länge des ersten Stutzens (6) über 15 cm und insbesondere 25 cm beträgt.
